(19) Europäisches Patentamt
European Patent Office
Office européen des brevets

(11) Publication number: **0 126 722**
**A2**

# (12) EUROPEAN PATENT APPLICATION

(21) Application number: **84850155.7**

(51) Int. Cl.³: **C 12 M 1/00**

(22) Date of filing: **17.05.84**

(30) Priority: **23.05.83 GB 8314250**

(43) Date of publication of application: **28.11.84**
**Bulletin 84/48**

(84) Designated Contracting States: **BE DE FR IT NL SE**

(71) Applicant: **BIOSYSTEM E AB, P.O. Box 24,**
**S-351 03 Växjö (SE)**

(72) Inventor: **Pola, Antonin, Biskopsvägen 51, S-352 39 Växjö**
**(SE)**

(74) Representative: **Franzén, Lars Hjalmar et al,**
**AWAPATENT AB Box 5117, S-200 71 Malmö (SE)**

## (54) Plant for processing organic material.

(57) A plant for processing organic material (18) which contains liquid and solid phases (19, 20), has a separator (6) for separation of the contaminated liquid and solid phases (19, 20) from one another, the contaminated liquid phase (19) being conducted to a recirculator (52) connected to an anaerobic filter (30) for purification of the contaminated liquid phase (19). In addition to the contaminated liquid phase (19) the recirculator (52) also receives the liquid phase (41) purified in the anaerobic filter (30), so that the contaminated liquid phase (19) and the purified liquid phase (41) are mixed in desired proportions for introduction into the filter (30). The remainder of the purified liquid phase (41) is withdrawn and can be conducted to say a recipient. The contaminated solid phase (20) is conducted from the separator (6) to a biogas reactor and after stabilisation to a thickener/drier for conversion into a sludge (77) which is useful for landfilling, deposition, composting, fertilizing and like purposes. The liquid (78) remaining in the thickener/drier is conducted to the recirculator (52) for admixture with the mixture (40). The biogas (45, 73) generated in the anaerobic filter (30) and the biogas reactor, respectively, is collected for use.

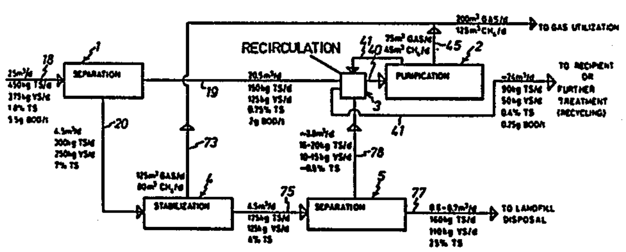


EP 0 126 722 A2

# PLANT FOR PROCESSING ORGANIC MATERIAL

This invention relates to a plant for processing organic material, preferably pig manure and the like, which contains liquid and solid phases.

Pig farms having average and big pig populations yield very large amounts of manure which for the greater part contains a liquid phase (urine), the remainder being a solid phase (droppings) which includes bedding and the like. Such manure has to be taken care of and can thus be processed in a biogas reactor in which anaerobic bacteria are added to the manure to generate biogas which mainly contains methane and can be used as an energy source for the operation of gas boilers, engines, power supply units etc. and for the formation of digested sludge which is a useful landfill material and/or fertilizer.

Owing to the large amounts of manure the biogas reactor with the pertaining peripheral equipment has to be given a corresponding size, which necessitates large economical and structural investments.

Moreover, the biogas reactor because of the large proportion of liquid phase in the manure is not as highly effective as would be desirable, since a complete treatment of the manure will require an unacceptably long dwell time.

For greater efficiency it would therefore be possible to carry out the treatment in the thermophilic temperature range at about 45-60$^{o}$C, but this treatment is sensitive to stoppages and requires a costly heating of the manure.

Therefore, the object of the present invention is to provide a plant which is of a novel and efficiency increasing construction to eliminate the drawbacks outlined in the foregoing.

According to the invention, this plant comprises an anaerobic filter for purification of the contaminated liquid phase, and a recirculator for receiving the contaminated liquid phase and the liquid phase purified in the filter and for mixing, according to need, the contaminated liquid phase with a large or small quantity of the purified liquid phase, said mixture being supplied to the filter and the remainder of the purified liquid phase being conducted to say a recipient. The plant further comprises a separator for separating the contaminated liquid and solid phases from one another, the contaminated liquid phase being led, as mentioned above, to the recirculator, while the contaminated solid phase is discharged for processing preferably to a biogas reactor, the biogas generated in the reactor being collected for use, while the solid phase treated in the reactor may be supplied to a thickener/drier for conversion into a sludge for land filling, deposition, composting, fertilizing and like purposes.

A specially preferred embodiment of a processing plant according to the invention will now be described in the following with reference to the accompanying drawings. In the drawings:

Fig. 1 is a flow diagram of the plant;

Fig. 2 is a side view of a separator comprised in the plant;

Fig. 3 is a cross-section taken on line III-III in Fig. 2;

Fig. 4 is a top plan view of a recirculator comprised in the plant;

Fig. 5 is a section on line V-V in Fig. 4;

Fig. 6 is a side view of an anaerobic filter of the plant;

Fig. 7 is a cross-section on line VII-VII in Fig. 6; and

Figs. 8 and 9 are sections on lines VIII-VIII and IX-IX, respectively, in Fig. 7.

The plant according to the invention for processing pig manure, which is illustrated by way of a flow diagram in Fig. 1, comprises five main stages, viz.: separation 1, purification 2, recirculation 3, stabilisation 4 and separation 5.

The separation stage 1 includes a separator 6, see Figs. 2 and 3, which comprises an upright container 8 placed on the ground 7 and having a cylindrical circumferential wall 9, a roof 10 and a conically tapering bottom 11. In the embodiment illustrated, the container 8 is placed on columns 12 which are embedded or otherwise fixed in a foundation plate 13 on the ground 7 or in cesspit walls.

In the roof 10 the container 8 has an inlet 14 which is connected via a conduit 15 to a submersible pump 16. The pump 16 is placed on the bottom of a cesspit 17 in which manure 18 is collected from one or more pig houses (not shown). The manure 18 in the cesspit 17 is pumped by means of the pump 16 through the conduit 15 and inlet 14 to the container 8 of the separator 6, in which container the contaminated liquid and solid phases 19 and 20, respectively, are separated from one another by sedimentation. On overcharging of the container 8, if any, the excess manure can be returned to the cesspit 17 through a conduit 21.

The separated contaminated solid phase 20 is discharged through an outlet 22 at the bottom 11 of the container 8 and is supplied by means of a pump 23 and through a conduit 24 to the stabilisation stage 4 for treatment in a manner to be described more in detail below.

The contaminated liquid phase 19 in turn is led through an outlet 25 and a conduit 26 to the recirculation and purification stages 3, 2 for treatment in a manner to be described more in detail below. In the embodiment according to Fig. 2, the outlet 25 has several vertically spaced points 27 for tapping of the container 8. These tapping points are controllable by means of valves 28 so as to open and close for withdrawal of the contaminated liquid phase 19 at different levels of the container 8 as close to the surface 29 in the container 8 as possible, depending on the quantity filled thereinto. Withdrawing the contaminated liquid phase 19 in this manner, one obtains a calm laminar flow of said phase to the tapping point 27 concerned.

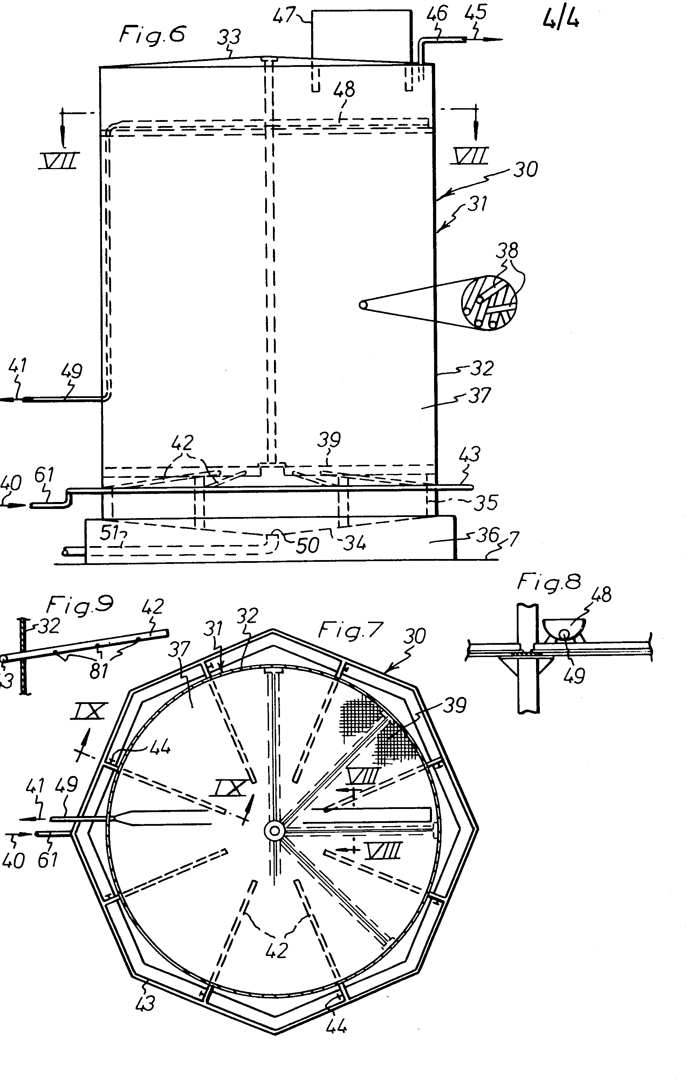

The purification stage 2 includes an anaerobic filter 30 for purification of the contaminated liquid phase 19. The filter 30 is of the continuously operating type and comprises an upright container 31 having a cylindrical circumferential wall 32, a roof 33 and a bottom 34. The container 31 is placed on columns 35 which in turn are embedded or otherwise fixed in a foundation 36 on the ground 7. The container 31 of the anaerobic filter 30 contains a bed 37 of hollow or solid, relatively large bodies 38 carrying anaerobic bacteria on their surfaces, which bacteria form a so-called bioskin on said surfaces. As will appear from the magnification to the right in Fig. 6, the bodies 38 are rod-shaped, but it should be pointed out that the bodies can have any optional shape. The bed 37 is supported on a netting 37 of expanded metal, which is spaced some distance upwardly from the bottom 34 of the container 31, as will appear from Fig. 6.

The anaerobic filter 30 is supplied with a mixture 40, to be described more in detail below, of contaminated liquid phase 19 and already purified liquid phase 41. The supply of the mixture 40 takes place beneath the bed 37 and is brought about by means of several supply pipes 42 uniformly distributed around the container 31 and having their one ends connected to a manifold 43 which is disposed around the container 31. As will appear from Figs. 6 and 9, said supply pipes 42 extend obliquely upwardly towards the center of the container 31, the inclination amounting to about 1:10 in the case illustrated. Where the supply pipes 42 are connected to the manifold 43 there are arranged manually or automatically operated valves 44 for connection of an optional number of supply pipes 42. To dispense the mixture 40 the supply pipes 42 have several downwardly opening outlets 81, see Fig. 9.

When urged in an upward direction through the bed 37 the mixture 40 is purified by the action of the anaerobic bacteria on the bodies 38, the biogas 45 generated collecting in the upper portion of the container 31 and being discharged for use through a gas pipe 46.

A manhole 47 with water seal is provided in the roof 33 of the container 31 to permit inspection of the interior of the container. In addition to being a gas seal the water seal also serves as a safety valve against excess pressure in the container 31.

Withdrawal of the liquid phase 41 purified in the bed 37 takes place, as will appear from Fig. 6, over the bed by means of a collecting trough 48, see also Fig. 8, which is horizontally placed directly above the bed 37, in the embodiment illustrated. The collecting trough 48 is open in an upward direction and connected at one end via a water seal (not shown) to a discharge pipe 49 for the purified liquid phase 41.

At the lowermost point of the bottom 34 there is arranged an outlet 50 which is connected to the earlier described cesspit 17 through a conduit 51 and which serves to clean the anaerobic filter 30.

As earlier mentioned, there is a recirculation stage 3 between the separation stage 1 and the purification stage 2. In the embodiment illustrated, said recirculation stage includes a recirculator 52. The recirculator has a container 53 of rectangular cross-section and is preferably made of concrete with side walls 54 and a bottom 55. The container 53 is also placed on the ground 7, either directly or via a foundation (not shown). The container 53 is divided into two chambers 56, 57 by means of a partition 58 between two opposite side walls 54. The first chamber 56 in one of the side walls 54 has an inlet 59 which is connected via the conduit 26 to the outlet 25 of the separator 6 so as to receive the contaminated liquid phase 19 therefrom. In the side wall 54 of the first chamber 56 which is opposed to the inlet 59 there is provided an outlet 60 which is connected via a feed pipe 61 to the manifold 43 of the anaerobic filter 30 for supply of the mixture 40. A feed pump 62 is installed in the feed pipe 61 for positive feed of the mixture 40 to the filter 30. A protective netting 63 is arranged around the outlet 60 to prevent solid impurities, if any, from entering the filter 30. The second chamber 57 has an inlet 64 which is connected via the discharge pipe 49 to the collecting trough 48 of the anaerobic filter 30 so as to receive the purified liquid phase 41 therefrom. A check valve 65 with a flap 66 is inserted in the partition 58 and opens towards the first chamber 56. Said check valve permits the purified liquid phase coming from the filter 30 through the inlet 64 to flow from the second chamber 57 to the first chamber 56, but prevents a flow from the first chamber 56 to the second chamber 57.

The check valve 65 is of such a design that say two thirds of the purified liquid phase 41 from the filter 30 flow into the first chamber 56 to mix with the contaminated liquid phase 19 coming from the separator 6 through the inlet 59 so that the mixture 40 is formed, which is to be supplied to the anaerobic filter 30. The remainder of the purified liquid phase 41 is conducted via an outlet 67 in the side wall 54 of the second chamber 57 which is opposed to the inlet 64, and via a conduit 68 to a recipient (not shown) or to for example one or more aerobic filters for further treatment therein, if considered necessary.

An overflow 69 is arranged between the inlet 64 and the outlet 67 of the second chamber 57 to provide the desired flow of the purified liquid phase 41 from the second chamber 57 to the first chamber 56 through the check valve 65. Said overflow extends between the partition 58 and the opposite side wall 54 and has its upper edge 70 placed on a higher level than the check valve 65, as will appear from Fig. 5.

To prevent overcharging of the first chamber 56 a further outlet 71 is provided in one of the side walls 54 of that chamber, said outlet 71 being connected to the cesspit 17 through a conduit 72.

The stabilisation stage 4 includes a biogas reactor for stabilising treatment of the contaminated solid phase 20 coming from the separator 6 through the conduit 4. The biogas reactor is of conventional construction and function and as it is not in itself part of the present invention, its construction and function will not be described more in detail. The biogas 73 generated in the biogas reactor is collected in a similar manner as the biogas 45 from the anaerobic filter 30 and is conducted for this purpose through a conduit to a place of use. The solid phase 75 stabilised in the biogas reactor is fed through a conduit to a thickener/drier, for example a sludge press, comprised in the separation stage 5. This thickener/drier is also of conventional construction and function and need not therefore be described more in detail. In the thickener/drier the solid phase 75 is converted into a sludge 77 which can be used for various purposes, for example landfilling, deposition, composting, fertilizing etc. The excess liquid 78 from the thickening/drying process is conducted through a conduit 79 to the recirculator 52, more precisely the first chamber 56 thereof, via an inlet 80 to permit mixing this liquid with the earlier mixture 40 of contaminated liquid phase 19 and purified liquid phase 41.

A specifically preferred embodiment of the plant according to the invention has been illustrated and described in the foregoing, but nothing prevents the plant from being designed in another manner within the scope of the patent protection solicited. Neither is the plant necessarily restricted to the processing of pig manure, but in principle equally useful for processing other organic materials which contain liquid and preferably also solid phases.

CLAIMS

1. A plant for processing organic material (18), preferably pig manure and the like, which contains liquid and solid phases (19, 20), comprising an anaerobic filter (30) for purification of the contaminated liquid phase (19), and a recirculator (52) for receiving the contaminated liquid phase (19) and the liquid phase (41) purified in the filter (30) and for mixing, according to need, the contaminated liquid phase with a large or small quantity of the purified liquid phase, the mixture (40) being supplied to the filter (30) and the remainder of the purified liquid phase (41) being conducted to say a recipient.

2. A plant as claimed in claim 1, in which the biogas (45) generated in the filter (30) is collected for use.

3. A plant as claimed in claim 1 and 2, in which the filter (30) is of the continuously operating type and comprises a container (31) containing a bed (37) of hollow or solid, relatively large bodies (38) carrying anaerobic bacteria which form a skin on the surfaces of the bodies, the supply of the mixture (40) taking place beneath the bed (37) and the withdrawal of the purified liquid phase (41) taking place above the bed.

4. A plant as claimed in claim 3, in which the bodies are spherical, conical, cylindrical or rod-shaped and the bed (37) is supported by a netting (39) of expanded metal.

5. A plant as claimed in claims 3 and 4, in which the supply of the mixture (40) beneath the bed (37) is brought about by means of several uniformly distributed supply pipes (42) which are connected at their one ends to a manifold (42) arranged around the container (31) and connected to the recirculator (52), and which extend slightly obliquely upwards towards the center of the container (31), the supply pipes (42) having several downwardly opening outlet holes (81) for the mixture (40).

6. A plant as claimed in any one of claims 3-5, in which the withdrawal of the purified liquid phase (41) above the bed (37) is brought about by means of one or more substantially horizontally placed, upwardly open collecting troughs (48) which are connected at their one ends via a water seal to a discharge pipe (49) connected to the recirculator (52).

7. A plant as claimed in any one of the preceding claims, in which the recirculator (52) comprises a container (53) which is divided into at least two chambers (56, 57) with a partition (58) therebetween, the first chamber (56) having an inlet (59) for receiving the contaminated liquid phase (19)'and an outlet (60) to the filter (30), while the second chamber (57) has an inlet (64) from the filter (30) and an outlet (67) leading say to the recipient, and in which plant the partition (58) accommodates a check valve (65) opening towards the first chamber (56) to permit purified liquid phase (41) to flow from the second chamber to the first chamber (57, 56) so as to mix with the contaminated liquid phase (19) but to prevent any flow from the first chamber to the second chamber (56, 57).

8. A plant as claimed in claim 7, in which an overflow (69) is provided between the inlet and the outlet (64, 67) of the second chamber (57), and the upper edge (70) of said overflow is on a higher level than the check valve (65) in the partition (58) between the chambers (56, 57).

9. A plant as claimed in any one of the preceding claims, further comprising a separator (6) for separating the contaminated liquid and solid phases (19, 20) from one another, the contaminated liquid phase (19) being led to the recirculator (52) while the contaminated solid phase (20) is discharged for treatment.

10. A plant as claimed in claim 9, in which the separator (6) comprises a container (8) having an inlet (14) for supplying the contaminated liquid and solid phases (19, 20) from say a collection pit (17), an outlet (25) for conducting the contaminated liquid phase (19) to the recirculator (52), and an outlet (22) for discharging the contaminated solid phase (20).

11. A plant as claimed in claim 10, in which the outlet (25) for the contaminated liquid phase (19) has several vertically spaced points (27) for tapping of the container (8), said tapping points being controllable so as to open and close for withdrawal of the contaminated liquid phase (19) on different levels of the container, depending on the quantity filled thereinto, as close to the surface (29) as possible and so as to generate a laminar flow of the contaminated liquid phase to the tapping point (27) concerned.

12. A plant as claimed in any one of claims 9-11, in which the outlet (22) for the contaminated solid phase (20) is placed at the preferably conically tapering bottom (11) of the container (8).

13. A plant as claimed in any one of preceding claims, further comprising a biogas reactor for treatment of the contaminated solid phase (20) coming from the separator (6), the biogas (73) generated in the reactor being collected in a similar manner as the biogas (45) from the filter (30), while the solid phase (75) treated in the reactor is discharged for further treatment.

14. A plant as claimed in claim 13, in which the solid phase (75) treated in the reactor is conducted to a thickener/drier to be converted into a sludge (77) for landfilling, deposition, composting, fertilizing and like purposes, the excess liquid (78) from the thickening/drying process being conducted to the recirculator (52) for introduction into the filter (30) together with the unprocessed and purified liquid phases (19, 41).

15. A plant substantially as herein described with reference to the accompanying drawings.

Fig.1

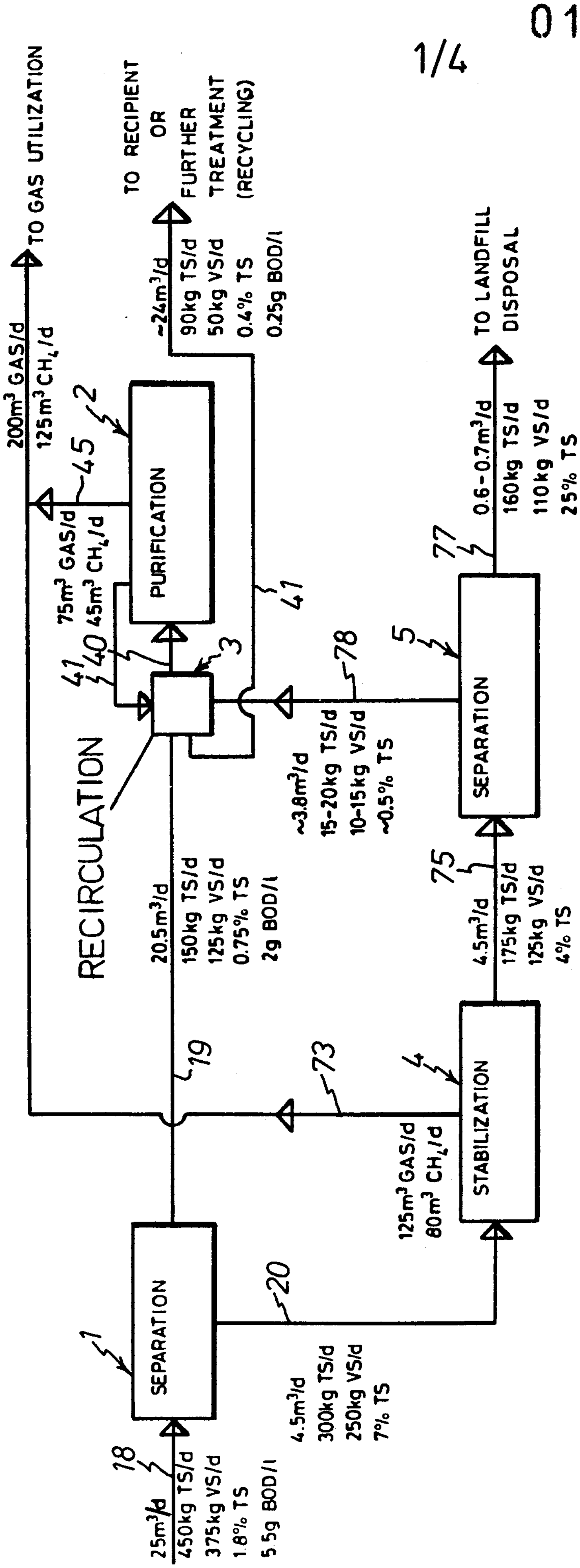
TO GAS UTILIZATION
TO RECIPIENT OR FURTHER TREATMENT (RECYCLING)
TO LANDFILL DISPOSAL
PURIFICATION
SEPARATION
STABILIZATION
SEPARATION
RECIRCULATION
200m³ GAS/d
125m³ CH₄/d
75m³ GAS/d
45m³ CH₄/d
~24m³/d
90kg TS/d
50kg VS/d
0.4% TS
0.25g BOD/l
0.6-0.7m³/d
160kg TS/d
110kg VS/d
25% TS
~3.8m³/d
15-20kg TS/d
10-15kg VS/d
~0.5% TS
20.5m³/d
150kg TS/d
125kg VS/d
0.75% TS
2g BOD/l
4.5m³/d
175kg TS/d
125kg VS/d
4% TS
125m³ GAS/d
80m³ CH₄/d
4.5m³/d
300kg TS/d
250kg VS/d
7% TS
25m³/d
450kg TS/d
375kg VS/d
1.8% TS
5.5g BOD/l
1
2
3
4
5
18
19
20
40
41
45
73
75
77
78

Fig.2

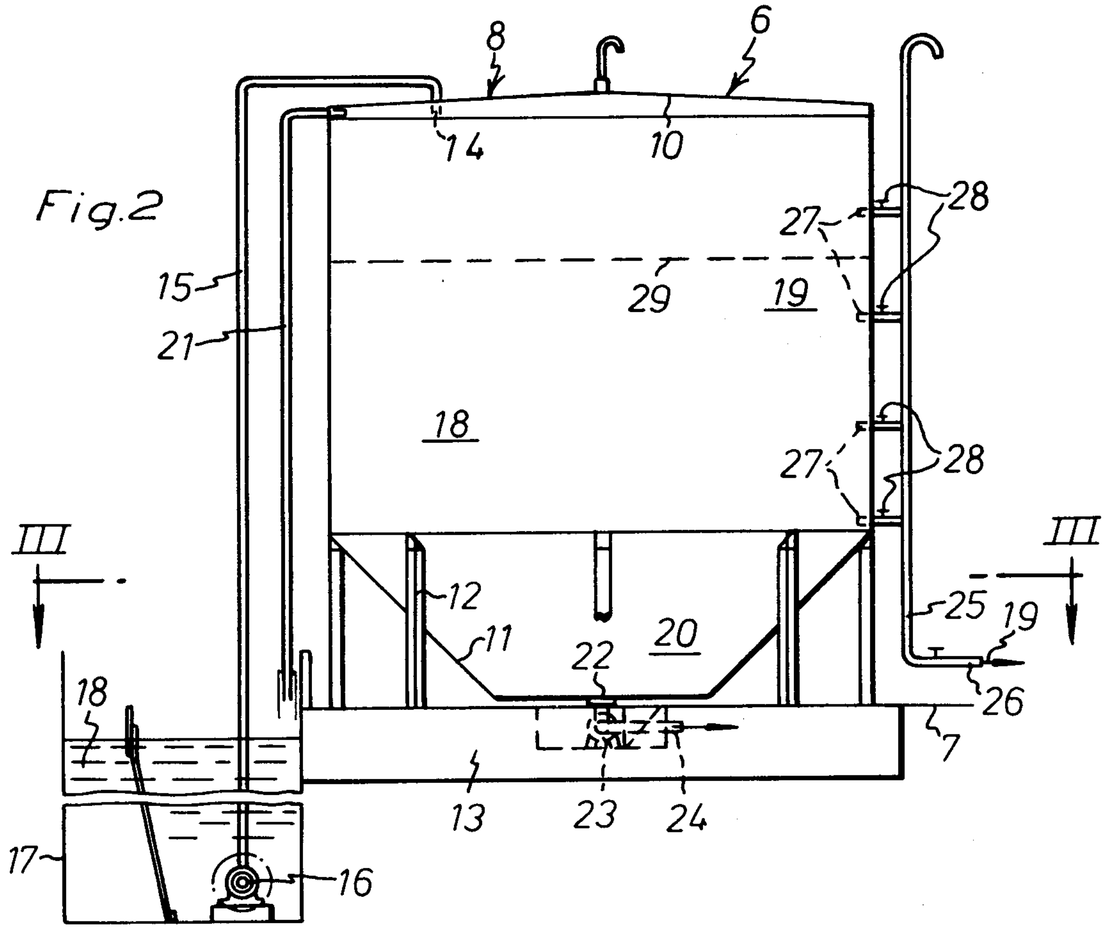
8
6
14
10
28
27
29
19
15
21
18
27
28
III
III
12
25
19
11
20
22
26
18
7
13
23
24
17
16

Fig.3

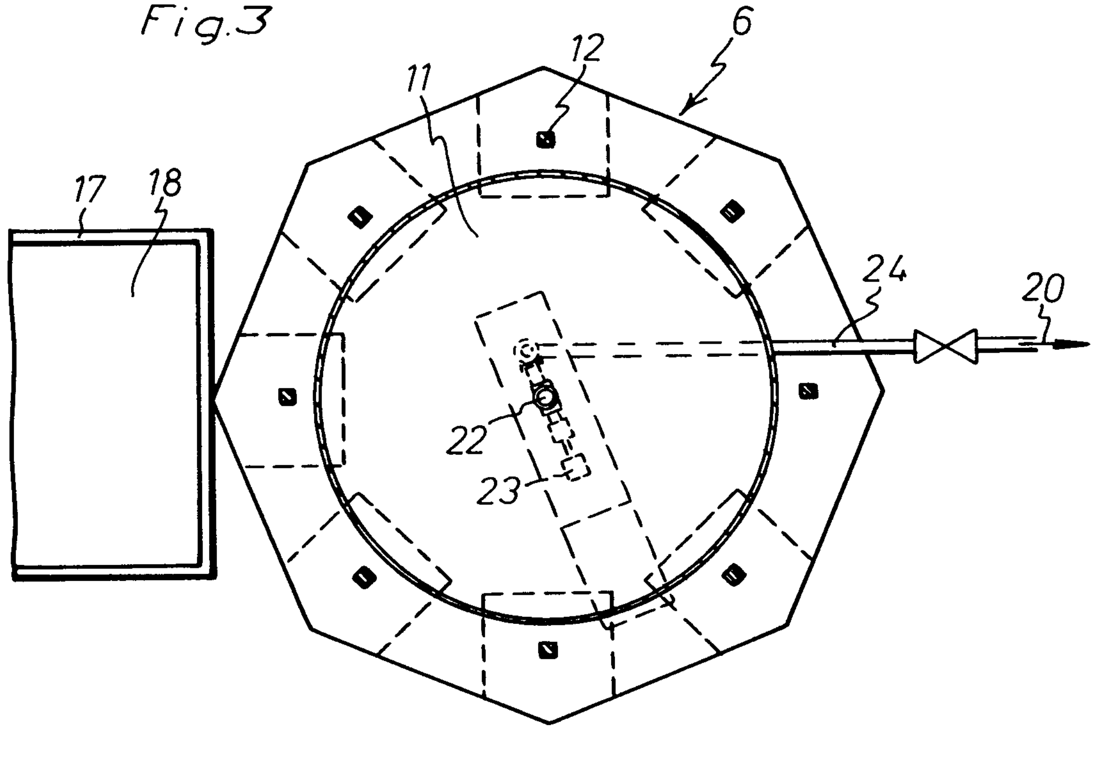
6
12
11
17
18
24
20
22
23

Fig.4

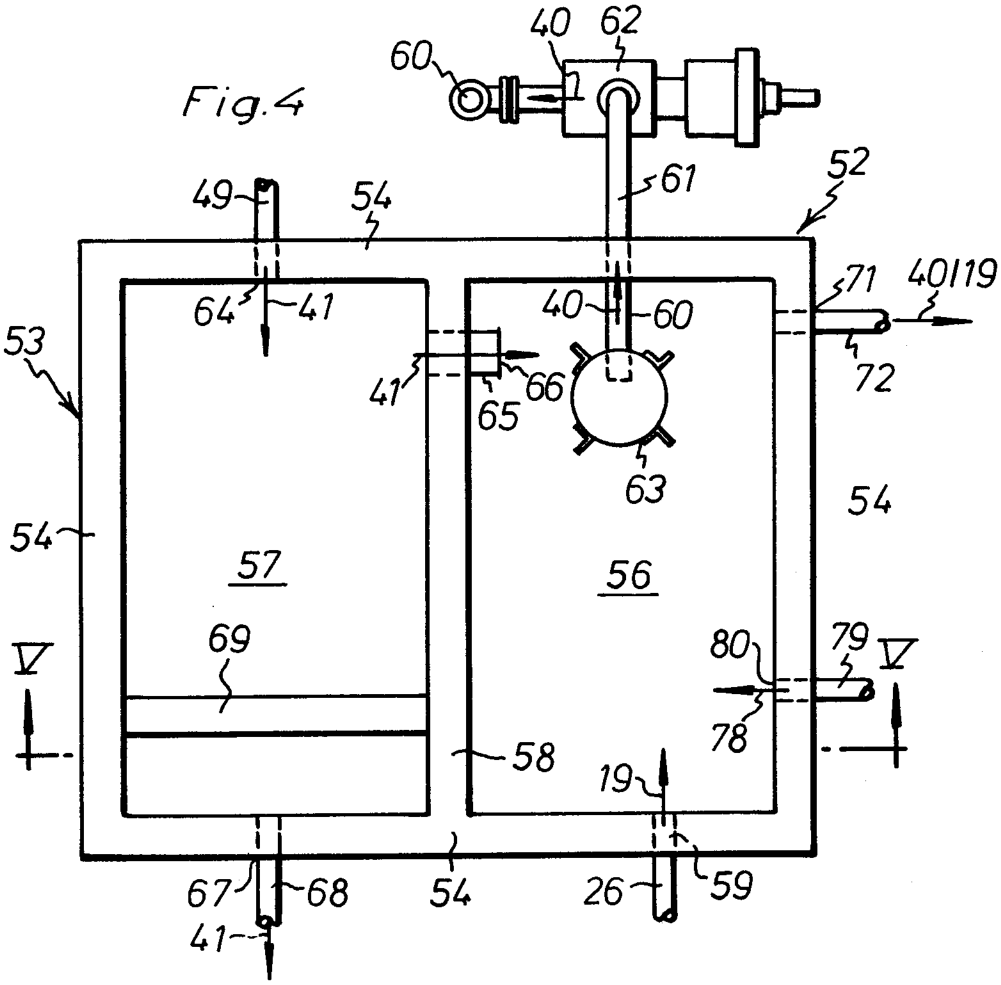
60
40
62
61
52
49
54
71
40119
64
41
40
60
53
41
66
65
72
63
54
54
57
56
69
80
79
V
V
78
58
19
67
68
54
26
59
41

Fig.5

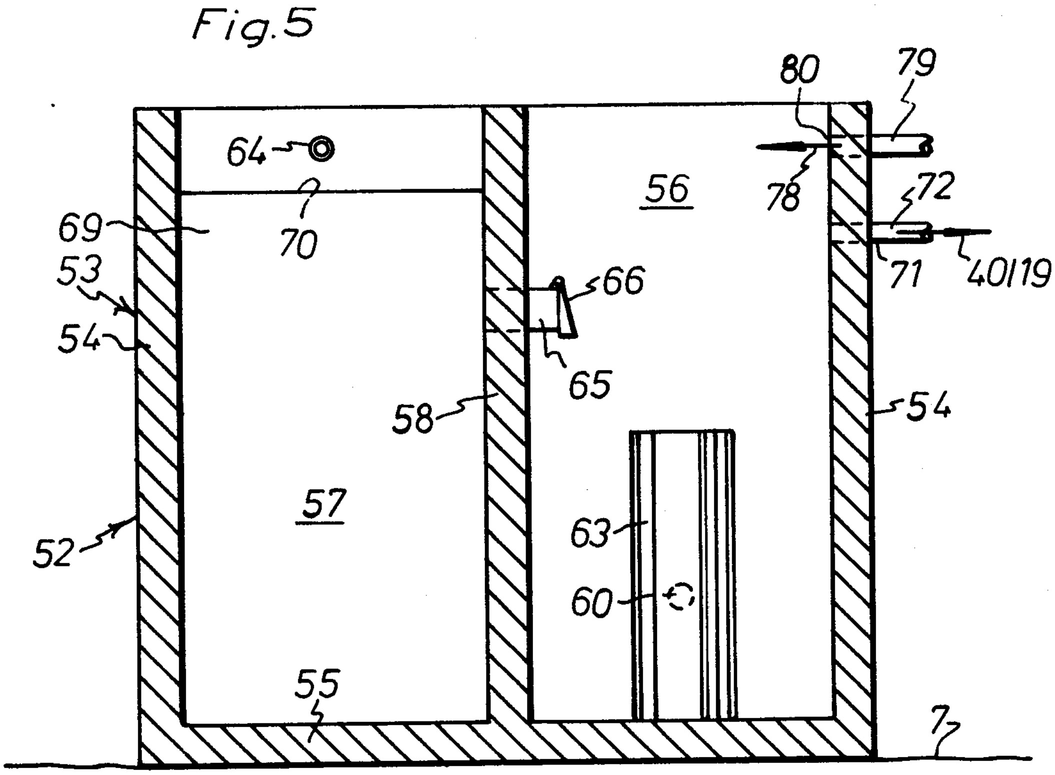
80
79
64
56
78
72
69
70
71
40119
53
66
54
65
58
54
57
63
52
60
55
7

Fig.6

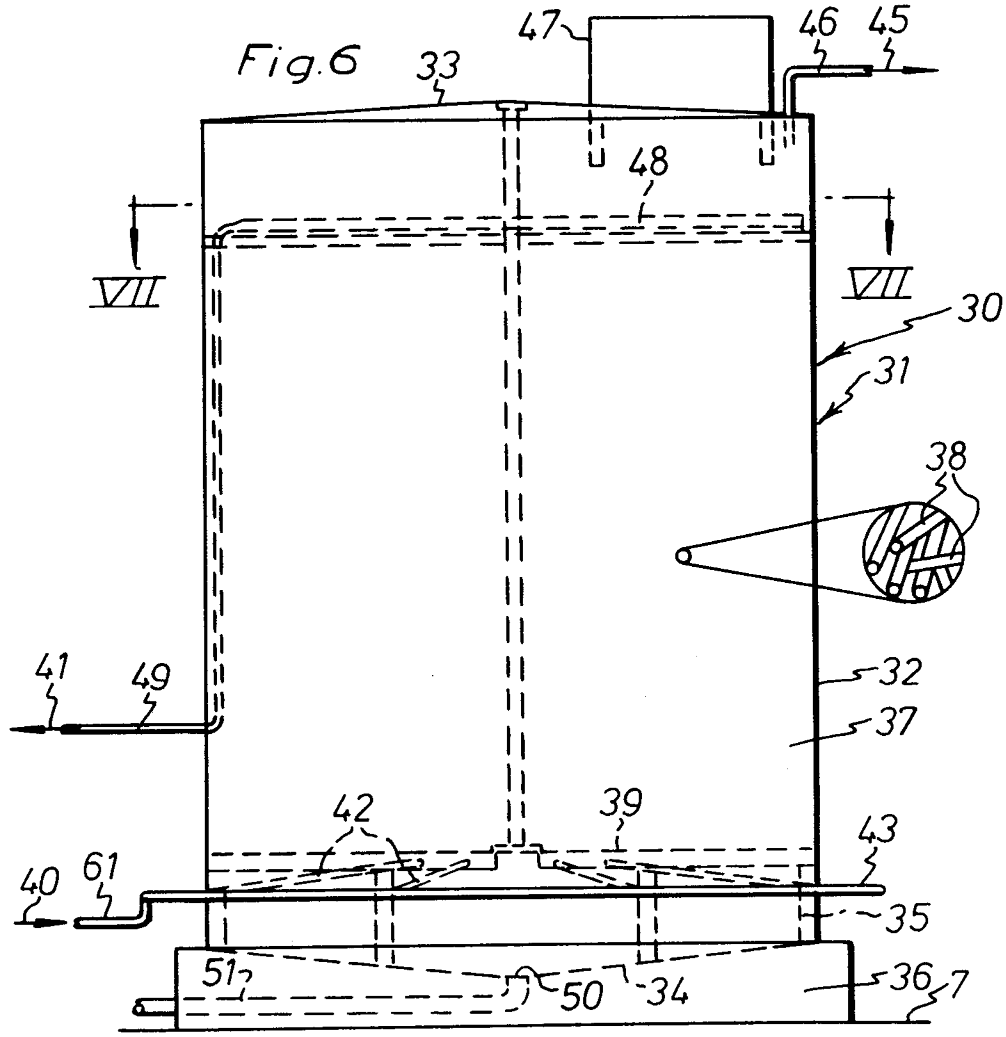


Fig.9

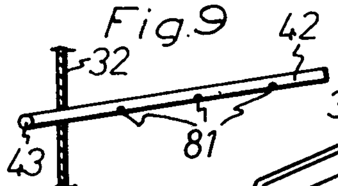


Fig.8

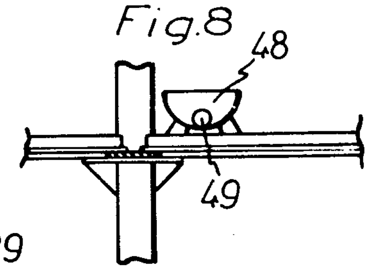


Fig.7